Europäisches Patentamt

European Patent Office

Office européen des brevets

(19)

(11) **EP 0 901 356 B1**

(12) **EUROPÄISCHE PATENTSCHRIFT**

(45) Veröffentlichungstag und Bekanntmachung des Hinweises auf die Patenterteilung:
**05.07.2000 Patentblatt 2000/27**

(51) Int Cl.⁷: **A61F 13/02**, C09J 5/08, C09J 7/02, C08J 9/12

(21) Anmeldenummer: **97921803.9**

(22) Anmeldetag: **26.04.1997**

(86) Internationale Anmeldenummer:
**PCT/EP97/02177**

(87) Internationale Veröffentlichungsnummer:
**WO 97/43992 (27.11.1997 Gazette 1997/51)**

(54) **SELBSTKLEBEND BESCHICHTETES, LUFTDURCHLÄSSIGES TRÄGERMATERIAL, VERFAHREN ZU DESSEN HERSTELLUNG UND VERWENDUNG**

AIR-PERMEABLE SUBSTRATE MATERIAL WITH A SELF-ADHESIVE COATING, PROCESS FOR ITS PRODUCTION AND ITS USE

MATERIAU SUPPORT PERMEABLE A L'AIR AVEC COUCHE AUTOCOLLANTE, SON PROCEDE DE FABRICATION ET SON UTILISATION

(84) Benannte Vertragsstaaten:
**AT DE ES FR GB IT NL SE**

(30) Priorität: **18.05.1996 DE 19620109**

(43) Veröffentlichungstag der Anmeldung:
**17.03.1999 Patentblatt 1999/11**

(73) Patentinhaber: **Beiersdorf Aktiengesellschaft 20245 Hamburg (DE)**

(72) Erfinder:
• **HIMMELSBACH, Peter**
  **D-21614 Buxtehude (DE)**
• **JAUCHEN, Peter**
  **D-22455 Hamburg (DE)**

(56) Entgegenhaltungen:
**FR-A- 2 319 699        JP-B- 50 004 993
US-A- 5 342 858**

**Beschreibung**

Die Erfindung betrifft selbstklebend ausgerüstete, luftdurchlässige Trägermaterialien, welche auf mindestens einer Seite vollflächig mit einer Heißschmelzselbstklebemasse beschichtet sind, ein Verfahren zu deren Herstellung sowie ihre Verwendung.

Heißschmelzselbstklebemassen auf Basis natürlicher und synthetischer Kautschuke und anderer synthetischer Polymere sind bekannt und werden zunehmend eingesetzt. Ihr wesentlicher Vorteil besteht darin, daß im Gegensatz zu den aus Lösung oder als wässrige Dispersion aufgebrachten Massen, keine aufwendigen und zum Teil umweltbelastenden Verfahren zum Entfernen der Lösungsmittel oder des Wassers notwendig sind.

Es wurde bereits vorgeschlagen derartige Selbstklebemassen, insbesondere auf Acrylat-Basis, für medizinische Produkte einzusetzen, wobei auch luftdurchlässige Gewebe oder Vliese als Trägermaterial genannt werden (US-PS 4 762 888, US-PS 4 879 178, EP-PS 436 159 und EP-PS 578 151). Ein Nachteil dieser vollflächig beschichteten Produkte ist jedoch ihre zu geringe Luft- und Wasserdampfdurchlässigkeit. Eine Verbesserung der Klebeeigenschaften läßt sich zudem meist nur mit einem höheren Masseauftrag erreichen.

Es ist ferner bekannt, derartige Selbstklebemassen nicht nur vollflächig sondern auch rasterpunktförmig, beispielsweise durch Siebdruck (DE-PS 42 37 252), wobei die Klebstoffpünktchen auch unterschiedlich groß und/oder unterschiedlich verteilt sein können (EP-PS 353 972), oder durch Tiefdruck in Längs- und Querrichtung zusammenhängenden Stegen aufzubringen (DE-PS 43 08 649).

Der Vorteil des rasterförmigen Auftrags besteht darin, daß die Klebematerialien bei entsprechend porösem Trägermaterial luft- und wasserdampfdurchlässig sowie in der Regel leicht wieder ablösbar sind.

Ein Nachteil dieser Produkte besteht jedoch darin, daß bei zu hoher Flächendeckung der an sich undurchlässigen Klebeschicht die Luft- und Wasserdampfdurchlässigkeit sich entsprechend verringert sowie der Klebemassenverbrauch steigt und bei geringer Flächendeckung der Klebeschicht die Klebeeigenschaften leiden, d.h. das Produkt löst sich zu leicht vom Untergrund.

Gemäß der WO 95/01408 werden spezielle elastomere, Heißschmelzklebeschäume auf Blockcopolymer-Basis als Kaschierkleber bei der Windelherstellung eingesetzt, wobei es jedoch nur auf deren elastische Eigenschaften ankommt.

Aufgabe der Erfindung war es deshalb, die genannten Nachteile zu vermeiden und ein Produkt bzw. Verfahren zu entwickeln, welches - bei entsprechend porösem Trägermaterial - eine sehr gute Luft- und Wasserdampfdurchlässigkeit sowie im allgemeinen gute Klebeeigenschaften bei geringem Klebemassenverbrauch aufweist.

Gelöst wird diese Aufgabe durch ein selbstklebend ausgerüstetes, luftdurchlässiges Trägermaterial mit einer mindestens auf einer Seite vollflächig aufgebrachten Heißschmelzklebemasse, das dadurch gekennzeichnet ist, daß die thermoplastische Klebemasse geschäumt ist und das Produkt bei einem Masseauftrag von mindestens 20 g/m$^2$, vorzugsweise 30 - 160 g/m$^2$, eine Luftdurchlässigkeit von mindestens 3 cm$^3$ / cm$^2$ /s aufweist.

Bewährt haben sich insbesondere Produkte mit einer Luftdurchlässigkeit von 3 - 150 cm$^3$ /cm$^2$ /s, bevorzugt 15 - 125 cm$^3$ /cm$^2$ /s und insbesondere 25 - 100 cm$^3$ /cm$^2$ / s.

Die Wasserdampfdurchlässigkeit sollte dabei mindestens 100g/m$^2$/ 24h, bevorzugt 100 - 5000 g/m$^2$ /24h und insbesondere 500 - 3000 g/m$^2$/ 24h betragen.

Je nach Trägermaterial und dessen Temperaturempfindlichkeit kann die Selbstklebeschicht direkt aufgetragen sein oder zuerst auf einen Hilfsträger aufgebracht und dann auf den endgültigen Träger transferiert werden. Auch ein nachträgliches Kalandern des beschichteten Produktes und/oder eine Vorbehandlung des Trägers, wie Coronabestrahlung, zur besseren Verankerung der Klebeschicht kann vorteilhaft sein.

Die Klebemassen werden bevorzugt mit inerten Gasen wie Stickstoff, Kohlendioxid, Edelgasen, Kohlenwasserstoffen oder Luft oder deren Gemischen geschäumt. In manchen Fällen kann sich auch ein Schäumen durch thermische Zersetzung gasentwickelnder Substanzen wie Azo-, Carbonat- und Hydrazid-Verbindungen als geeignet erweisen.

Der Schäumungsgrad, d.h. der Gasanteil, sollte mindestens etwa 10 Vol-% betragen und kann bis zu etwa 80% reichen. In der Praxis haben sich Werte von 30-70%, bevorzugt 50% Gasanteil gut bewährt. Wird bei relativ hohen Temperaturen von ca. 100° C und vergleichsweise hohem Innendruck gearbeitet, entstehen sehr offenporige Klebstoffschaumschichten, die besonders gut luft- und wasserdampfdurchlässig sind.

Als Selbstklebemassen lassen sich die bekannten thermoplastischen Heißschmelzklebemassen einsetzen auf Basis natürlicher und synthetischer Kautschuke und anderer synthetischer Polymere wie Acrylate, Methacrylate, Polyurethane, Polyolefine, Polyvinylderivate, Polyester oder Silikone mit entsprechenden Zusatzstoffen wie Klebharzen, Weichmachern, Stabilisatoren und anderen Hilfsstoffen soweit erforderlich. Ihr Erweichungspunkt sollte höher als 80° C liegen, da die Applikationstemperatur in der Regel mindestens 90° C beträgt, bevorzugt zwischen 120° und 150° C bzw. 180-220° C bei Silikonen. Gegebenenfalls kann eine Nachvernetzung durch UV- oder Elektronenstrahlen-Bestrahlung angebracht sein.

Besonders geeignet haben sich Selbstklebemassen auf Basis von A-B-A-Blockcopolymeren, welche aus harten und weichen Segmenten bestehen. Bevorzugt ist A ein Polymerblock basierend auf Styrol und B ein Polymerblock auf Basis von Ethylen, Propylen, Butylen, Butadien, Isopren oder deren Mischungen wie Ethylen/Butylen. Zusätzlich enthalten derartige Heißschmelzklebemassen in der Regel ein oder mehrere aliphatische oder aromatische Kohlenwasserstoffharze als Klebharze, ein oder mehrere mittel- oder langkettige Fettsäuren oder deren Ester sowie Stabilisatoren und gegebenenfalls andere Hilfsstoffe. Die Mengenbereiche der Bestandteile bewegen sich meist zwischen 15-70% Blockcopolymeren, 20-70% Klebharzen, 10-50% Weichmachern und geringen Mengen Stabilisatoren und anderen Hilfsstoffen.

Als Trägermaterialien können praktisch alle an sich porösen und luftdurchlässigen Träger, die üblicherweise für technische oder medizinische Zwecke verwendet werden, eingesetzt werden, d.h. Gewebe oder Gewirke, elastisch oder unelastisch, Kunststoffolien, Papiere, Vliese, Schaumstoffe oder Laminate daraus.

Die selbstklebend ausgerüsteten Trägermaterialien, die erfindungsgemäß mit einer geschäumten Heißschmelzselbstklebemasse beschichtet sind, zeichnen sich durch eine Reihe von Vorteilen aus. Durch die Schäumung der Klebemasse und die dadurch entstandenen offenen Poren in der Masse sind bei Verwendung eines an sich porösen Trägers die mit der Klebemasse beschichteten Produkte gut wasserdampf- und luftdurchlässig. Die benötigte Klebemassenmenge wird erheblich reduziert ohne Beeinträchtigung der Klebeeigenschaften. Die Klebemassen weisen eine überraschend hohe Anfaßklebrigkeit (tack) auf, da pro Gramm Masse mehr Volumen und damit Klebeoberfläche zum Benetzen des zu beklebenden Untergrundes zur Verfügung steht und die Plastizität der Klebemassen durch die Schaumstruktur erhöht ist. Auch die Verankerung auf dem Trägermaterial wird dadurch verbessert. Außerdem verleiht die geschäumte Klebebeschichtung den Produkten ein weiches und anschmiegsames Anfühlen.

Durch das Schäumen wird zudem die Viskosität der Klebemassen in der Regel gesenkt. Hierdurch wird die Schmelzenergie erniedrigt und es können auch thermoinstabile Trägermaterialien direkt beschichtet werden.

Die subjektiven Produktvorteile Anfaßklebrigkeit und Anschmiegsamkeit lassen sich unter Verwendung einer dynamisch-mechanischen Frequenzmessung gut quantifizieren. Hierbei wird ein schubspannungsgesteuertes Rheometer verwendet.

Die Ergebnisse dieser Meßmethode geben Auskunft über die physikalischen Eigenschaften eines Stoffes durch die Berücksichtigung des viskoelastischen Anteils. Hierbei wird bei einer vorgegebenen konstanten Temperatur der Schmelzhaftkleber zwischen zwei planparallelen Platten mit variablen Frequenzen und geringer Verformung (linear viskoelastischer Bereich) in Schwingungen versetzt. Über eine Aufnahmesteuerung wird computerunterstützt der Quotient (Q = tan δ) zwischen dem Verlust- (G'' viskoser Anteil) und dem Speichermodul (G' elastischer Anteil) ermittelt. Für das subjektive Empfinden der Anfaßklebrigkeit (Tack) wird eine hohe Frequenz und für die Anschmiegsamkeit eine niedrige Frequenz gewählt und die entsprechenden Quotienten über dem Schäumungsgrad ermittelt. Je höher der entsprechende Zahlenwert des Quotienten ist, desto besser ist die subjektive Eigenschaft.

Die Anfaßklebrigkeit und die Anschmiegsamkeit konnten erfindungsgemäß verbessert werden, wie in der Tabelle dargestellt.

$$Q = \tan \delta = G''/G'$$

| Bezeichnung | Anschmiegsamkeit niedrige Frequenz/RT | Anfaßklebrigkeit hohe Frequenz/RT |
|---|---|---|
| Schmelzhaftkleber A (nicht geschäumt) | $\tan \delta = 0,35 \pm 0,05$ | $\tan \delta = 0,45 \pm 0,05$ |
| Schmelzhaftkleber A Schaum Vol. ($N_2$)=50% | $\tan \delta = 0,46 \pm 0,05$ | $\tan \delta = 0,65 \pm 0,05$ |
| Schmelzhaftkleber A (nicht geschäumt) | $\tan \delta = 0,35 \pm 0,05$ | $\tan \delta = 0,45 \pm 0,05$ |
| Schmelzhaftkleber A Schaum Vol. ($N_2$) = 70% | $\tan \delta = 0,58 \pm 0,05$ | $\tan \delta = 0,88 \pm 0,05$ |
| Schmelzhaftkleber B (nicht geschäumt) | $\tan \delta = 0,05 \pm 0,03$ | $\tan \delta = 0,84 \pm 0,05$ |
| Schmelzhaftkleber B Schaum Vol. ($N_2$)=50% | $\tan \delta = 0,27 \pm 0,05$ | $\tan \delta = 1,15 \pm 0,05$ |
| Schmelzhaftkleber C (nicht geschäumt) | $\tan 8 = 0,06 \pm 0,03$ | $\tan \delta = 0,93 \pm 0,05$ |

(fortgesetzt)

| Bezeichnung | Anschmiegsamkeit niedrige Frequenz/RT | Anfaßklebrigkeit hohe Frequenz/RT |
|---|---|---|
| Schmelzhaftkleber C Schaum Vol. ($N_2$)=50% | $\tan \delta = 0{,}31 \pm 0{,}05$ | $\tan \delta = 1{,}25 \pm 0{,}05$ |

Es wurden verschiedene Schmelzhaftkleber, d.h. A auf Acrylat-, B und C auf Blockcopolymeren-Basis, gewählt und die Ergebnisse zeigen einen deutlichen Anstieg der $\tan \delta$-Werte durch die Schäumung, d.h. eine meßbar bessere Anschmiegsamkeit und Anfaßklebrigkeit.

Die geschilderten Vorteile machen die erfindungsgemäßen Trägermaterialien besonders geeignet für medizinische Zwecke. Daraus beispielsweise hergestellte Pflaster, Binden oder Bandagen oder zusätzlich mit einer Wundauflage versehene Wundverbände sind bei entsprechend ausgewähltem luftdurchlässigem Trägermaterial und einer hypoallergenen Klebemasse besonders gut hautverträglich, da sie über die ganze Fläche ausgeprägt luft- und wasserdampfdurchlässig sowie weich und anschmiegsam sind. Sie wirken wie gepolstert und weisen dadurch gute Trageeigenschaften auf bei gleichzeitig gutem Haftvermögen.

Die Produktvorteile durch das Schäumen der Klebemassen wie hohe Klebkraft sowie gute Luft- und Wasserdampfdurchlässigkeit können aus den folgenden Messungen entnommen werden.

1. Pflasterbinden mit geschäumten Hotmeltklebemassen kleben stärker auf der Haut als Pflasterbinden mit ungeschäumten Klebemassen bei gleichem Masseauftrag. Da die Klebkraft auf der Haut von Hauttyp zu Hauttyp unterschiedlich ist, wurde die ungeschäumte Klebemasse mit einem Index 100 benannt und die geschäumte Pflasterbinde in Relation gebracht. Dieses ergab folgende Ergebnisse:

Index = Klebkraft auf der Haut (geschäumt)/Klebkraft auf der Haut (ungeschäumt)

Tab.1:

| Klebkraftsteigerung auf Haut | | | |
|---|---|---|---|
| Masseauftrag | Gewebe (unelast.) | Gewebe (elast.) | Vlies |
| 40 g/m$^2$<br>60 g/m$^2$ | 105-110% | | 120-130% |
| 80 g/m$^2$<br>120 g/m$^2$ | 110-125%<br>120-170% | 110-120% | |

2. Pflasterbinden mit geschäumten Hotmeltklebemassen sind luftdurchlässiger als Pflasterbinden mit ungeschäumten Klebemassen bei gleichem Masseauftrag. Die Luftdurchlässigkeit von Pflasterbinden mit nicht geschäumten Klebemassen betrug bei den überprüften Mustern <1 cm$^3$/cm$^2$/sec. Die Luftdurchlässigkeiten beziehen sich auf einen Schäumungsgrad von 50% im Endprodukt.

Tab. 2:

| Luftdurchlässigkeit in Abhängigkeit vom Masseauftrag | | | | |
|---|---|---|---|---|
| Masseauftrag | Gewebe (unelast.) | Gewebe (elast.) ungedehnt | Gewebe (elast.) gedehnt | Vlies |
| 40 g/m$^2$<br>60 g/m$^2$ | 6-19 cm$^3$/cm$^2$/sec | | | 90-100 cm$^3$/cm$^2$/sec |
| 80 g/m$^2$<br>120 g/m$^2$ | 3 - 8 cm$^3$/cm$^2$/sec<br>0,5 - 3 cm$^3$/cm$^2$/sec | 20 - 35 cm$^3$/cm$^2$/sec | 90 - 110 cm$^3$/cm$^2$/sec | |

3. Die Luftdurchlässigkeit ist abhängig vom Schäumungsgrad. Die Ergebnisse wurden bei einem Masseauftrag von 80 g/m$^2$ ermittelt.

Tab. 3:

| Abhängigkeit der Luftdurchlässigkeit vom Schäumungsgrad | |
|---|---|
| Schäumunosgrad | unelast. Gewebe |
| 30% | 2 - 5 cm$^3$/cm$^2$/sec |

Tab. 3:   (fortgesetzt)

| Abhängigkeit der Luftdurchlässigkeit vom Schäumungsgrad ||
|---|---|
| Schäumunosgrad | unelast. Gewebe |
| 50% | 3 - 8 cm$^3$/cm$^2$/sec |
| 70% | 7 - 25 cm$^3$/cm$^2$/sec |

4. Weiterhin ist für die Haut die Durchlässigkeit für Wasserdampf von besonderer Bedeutung. Analoge Pflasterbinden mit ungeschäumten Klebemassen sind nicht wasserdampfdurchlässig. Die dargestellten Proben sind bei 23,5°C vorkonditioniert worden. Als Prüfparameter sind beispielhaft die Temperatur 37°C, der Sättigungsdampfdruck 6,274 kPa und die relative Luftfeuchtigkeit 30% genannt.

Tab. 4:

| Wasserdampfdurchlässigkeitsrate in Abhängigkeit vom Masseauftrag ||||
|---|---|---|---|
| Masseauftrag | Gewebe (unelast). in g/m$^2$ /24h | Gewebe (elast.) ungedehnt in g/m$^2$/24h | Vlies in g/m$^2$ /24h |
| 40 g/m$^2$ | 1020 | | |
| 60 g/m$^2$ | | | 2740 |
| 80 g/m$^2$ | 520 | 2510 | |
| 120 g/m$^2$ | 138 | | |

5. Im Folgenden wird die Abhängigkeit der Wasserdampfdurchlässigkeit vom Schäumungsgrad gezeigt. Hierfür wurde ein Masseauftrag von 80 g/m$^2$ gewählt. Die unter Punkt 4 beschriebenen Parameter sind konstant geblieben.

Tab. 5:

| Wasserdampfdurchlässigkeitsrate in Abhängigkeit vom Schäumungsgrad ||
|---|---|
| Schäumungsgrad | unelast. Gewebe in g/m$^2$ /24h |
| 30% | 240 |
| 50% | 520 |
| 70% | 990 |

Auch kohäsive Haftbeschichtungen, d.h. Antirutschbeschichtungen, welche nur auf sich selbst kleben bzw. kaum klebenden Charakter aufweisen, können erfindungsgemäß hergestellt werden.

Die vorteilhaften Eigenschaften der erfindungsgemäßen Klebebeschichtungen, wie geringer Klebstoffverbrauch, hohe Anfaßklebrigkeit und gute Anschmiegsamkeit auch an unebenen Flächen durch die Elastizität und Plastizität der geschäumten Klebemassen, lassen sich auch auf rein technischem Gebiet nutzen. Die erhaltenen Selbstklebebänder und andere auf diese Weise selbstklebend ausgerüsteten Produkte lassen sich vielfältig einsetzen.

Ein besonders geeignetes Verfahren zur Herstellung der erfindungsgemäß selbstklebend ausgerüsteten Trägermaterialien arbeitet nach dem Schaum-Mix-System. Hierbei wird der thermoplastische Haftkleber unter hohem Druck bei ca. 120 Grad Celsius mit trockenen Gasen wie z.B. Stickstoff, Luft oder Kohlendioxid in unterschiedlichen Volumenanteilen (etwa 10-80%) in einem Stator/Rotorsystem umgesetzt. Während der Gasvordruck >100 bar ist, betragen die Mischdrucke Gas/Thermoplast im System 40-100 bar, bevorzugt 40-70 bar. Der so hergestellte Haftklebeschaum gelangt über eine Leitung zum Düsenauftragswerk einer Schmelzbeschichtungsanlage.

Nachfolgend wird die Erfindung durch Beispiele näher erläutert.

Beispiel 1

Für die in der Orthopädie üblichen funktionalen Tapeverbände werden relativ starre, unelastische Gewebe eingesetzt. Tapeverbände werden zur Immobilisation des Bewegungsapparates und als Stützverbände u.a. zur Prophylaxe, Erstversorgung, Therapie und Rehabilitation angewendet. Hierzu werden starre Trägermaterialien mit einer Höchstzugkraft von ca. 60 N/cm und einer Höchstzugkraft-Dehnung von kleiner 20% einseitig mit einer Klebemasse beschichtet.

Erfindungsgemäß wurde eine geschäumte Hotmeltklebemasse auf Basis eines Blockcopolymeren verwendet. Hierzu wurden starre Gewebe mit ca. 40, 80, und 120 g/m$^2$ beschichtet. Alle Muster kleben auf der Haut und sind luftdurchlässig. Je größer der Klebemassenauftrag desto stärker klebt die Binde auf der Haut. Die Luftdurchlässigkeit nimmt mit steigendem Masseauftrag ab. Die mit 40 g/m$^2$ Klebemasse ausgerüstete Binde zeigte die beste Wasserdampfdurchlässigkeit. Der Schäumungsgrad betrug ca. 50%.

Hierdurch konnte gezeigt werden, daß die Luft- und Wasserdampfdurchlässigkeit funktional von dem Masseauftrag abhängen.

Weiter wurden starre Gewebe mit unterschiedlichem Schäumungsgrad hergestellt. Dabei zeigt sich der funktionale Zusammenhang der Luft- und Wasserdampfdurchlässigkeit in Abhängigkeit vom Schäumungsgrad. Je höher der Gasanteil in der applizierten Klebemasse ist, desto größer die Durchlässigkeit. Produkte mit einem Schäumungsgrad von 70% wiesen in der Versuchsreihe die höchste Durchlässigkeit auf (Tab. 3 und Tab. 5).

Muster mit der gleichen Klebemasse, welche jedoch ungeschäumt ist, weisen keine oder deutlich geringere Durchlässigkeiten auf.

Beispiel 2

Elastische Binden werden bisher in der Regel indirekt beschichtet. Hierbei wird die Klebemasse auf silikonbeschichtetes Trennpapier ausgestrichen und das Lösemittel im Trockenkanal entfernt. Anschließend wird das elastische Trägermaterial, ein Gewebe oder Gewirke, aufkaschiert. Eine derart hergestellte Binde ist jedoch nicht immer ausreichend luftdurchlässig.

Eine erfindungsgemäß hergestellte Binde wurde im Heißschmelzbeschichtungsverfahren mit einer mit Stickstoff geschäumten Klebemasse auf Basis eines Blockcopolymeren mit einem Masseauftrag von ca. 80 g/m$^2$ beschichtet. Bei dem Blockcopolymeren handelt es sich um ein Styrol-Ethylen-Butylen-Styrol-Blockcopolymerisat, welches mit Paraffinkohlenwasserstoffen versetzt worden ist. Das Verhältnis war ein Teil Polymer zu einem Teil Paraffinkohlenwasserstoff. Zu der hergestellten Mischung wurden 10% Polystyrolharz (z.B. Amoco 18240) gegeben. Der Kleber enthielt ferner ein Prozent Alterungsschutzmittel (β-(3,5 Di-t.butyl-4-hydroxy-phenyl)-propionsäure-n-octadecylester), Handelsname IRGANOX 1076) und weitere Kohlenwasserstoffharze und Fettsäureester, welche im Gesamtkleber nur zu geringen Mengen enthalten sind. Der Kleber ist handelsüblich erhältlich ( Fa. Füller). Die Klebemasse wurde nach dem oben beschriebenen Verfahren bei 120° C im Verhältnis 1:2 mit Stickstoff geschäumt. Der daraus resultierende Gasanteil im Endprodukt betrug dann 50%.

Die Luftdurchlässigkeit der Binde betrug im ungedehnten Zustand 20 - 35 cm$^3$/cm$^2$/s und im gedehnten Zustand ca. 100 cm$^3$/cm$^2$/s. Die Wasserdampfdurchlässigkeit war größer als 500 g/m$^2$/24h. Durch die geschäumte, teilweise offenporige Klebemasse ist die Binde auch in einem mehrlagigen Verband luft- und wasserdampfdurchlässig. Sie wird für Kompressions-, Stütz- und Entlastungsverbände eingesetzt, wobei die hohe Sofort- und Dauerklebekraft vorteilhaft sind. Weiter unterstützt die Klebemasse auf Basis des Blockcopolymers durch die Elastizität des Klebers die kompressive Wirkung der Binde. Die Modellierbarkeit und Anwenderempfindung sind durch die Schäumung der Klebemasse verbessert worden.

## Patentansprüche

1. Selbstklebend ausgerüstetes, luftdurchlässiges Trägermaterial mit einer mindestens auf einer Seite vollflächig aufgebrachten Heißschmelzselbstklebemasse, dadurch gekennzeichnet, daß die thermoplastische Klebemasse geschäumt ist und das Produkt bei einem Masseauftrag von mindestens 20 g/m$^2$ eine Luftdurchlässigkeit von mindestens 3 cm$^3$/cm$^2$/s aufweist.

2. Selbstklebend ausgerüstetes Trägermaterial gemäß Anspruch 1, dadurch gekennzeichnet, daß es eine Luftdurchlässigkeit von 3 - 150cm$^3$/cm$^2$/s, bevorzugt 15 - 125 cm$^3$/cm$^2$/s, insbesondere 25 - 100 cm$^3$/cm$^2$/s aufweist.

3. Selbstklebend ausgerüstetes Trägermaterial gemäß Anspruch 1 oder 2, dadurch gekennzeichnet, daß der Klebemassenauftrag 30 - 200 g/m$^2$, vorzugsweise 30 - 160 g/m$^2$ beträgt.

4. Selbsklebend ausgerüstetes Trägermaterial gemäß einem oder mehreren der vorangehenden Ansprüche, dadurch gekennzeichnet, daß es eine Wasserdampfdurchlässigkeit von mindestens 100 g/m$^2$/24 h aufweist.

5. Selbstklebend ausgerüstetes Trägermaterial gemäß Anspruch 4, dadurch gekennzeichnet, daß es eine Wasserdampfdurchlässigkeit von 100 - 5000g/m$^2$ /24 h, bevorzugt 500 - 3000g/m$^2$ /24 h aufweist.

6. Selbstklebend ausgerüstetes Trägermaterial gemäß einem oder mehreren der vorangehenden Ansprüche, dadurch gekennzeichnet, daß die Klebemasse mit einem inerten Gas wie Stickstoff, Kohlendioxid, Edelgasen, Kohlenwasserstoffe oder Luft oder deren Gemische geschäumt ist.

**7.** Selbstklebend ausgerüstetes Trägermaterial gemäß einem oder mehreren der vorangehenden Ansprüche, dadurch gekennzeichnet, daß der Gasanteil in der Klebemasse 10-80 Volumenprozent, vorzugsweise etwa 30-70%, beträgt.

**8.** Selbstklebend ausgerüstetes Trägermaterial gemäß einem oder mehreren der vorangehenden Ansprüche, dadurch gekennzeichnet, daß die Klebemasse auf A-B-A Blockcopolymer-Basis aufgebaut ist, wobei A Polystyrol und B Ethylen, Propylen, Butylen, Butadien, Isopren oder deren Mischungen wie Ethylen/Butylen bedeuten.

**9.** Verfahren zur Herstellung von selbstklebend ausgerüstetem Trägermaterial gemäß einem oder mehreren der vorangehenden Ansprüche, dadurch gekennzeichnet, daß die Heißschmelzselbstklebemassen unter hohem Druck mit inerten Gasen in Volumenanteilen von 10-80% in einem Stator/Rotorsystem umgesetzt werden, der so hergestellte Haftklebeschaum in die Düsenzuführung einer Schmelzbeschichtungsvorrichtung geleitet wird und von dieser auf das Trägermaterial appliziert wird.

**10.** Verwendung eines selbstklebend ausgerüsteten Trägermaterials gemäß einem oder mehreren derAnsprüche 1 bis 7 für medizinische Produkte.

**11.** Verwendung gemäß Anspruch 10, wobei die medizinischen Produkte Heftpflaster, Fixierpflaster, Testpflaster, Wundschnellverbände, Binden, orthopädische Binden und Bandagen, Wundauflagen, Inzisionsfolien oder Colostomiebeutel sind.

**Claims**

**1.** Air-permeable carrier material having a self-adhesive finish and a hotmelt self-adhesive composition applied to the entire area of at least one side, characterized in that the thermoplastic adhesive composition is foamed and the product has an air permeability of at least 3 $cm^3/cm^2/s$ for an amount applied of at least 20 $g/m^2$.

**2.** Carrier material having a self-adhesive finish according to Claim 1, characterized in that it has an air permeability of 3 - 150 $cm^3/cm^2/s$, preferably 15 - 125 $cm^3/cm^2/s$, in particular 25 - 100 $cm^3/cm^2/s$.

**3.** Carrier material having a self-adhesive finish according to Claim 1 or 2, characterized in that the amount of adhesive composition applied is 30 - 200 $g/m^2$, preferably 30 - 160 $g/m^2$.

**4.** Carrier material with a self-adhesive finish according to one or more of the preceding claims, characterized in that it has a water-vapour permeability of at least 100 $g/m^2/24$ h.

**5.** Carrier material having a self-adhesive finish according to Claim 4, characterized in that it has a water-vapour permeability of 100 - 5000 $g/m^2/24$ h, preferably 500 - 3000 $g/m^2/24$ h.

**6.** Carrier material having a self-adhesive finish according to one or more of the preceding claims, characterized in that the adhesive composition is foamed using an inert gas such as nitrogen, carbon dioxide, noble gases, hydrocarbons or air, or mixtures thereof.

**7.** Carrier material having a self-adhesive finish according to one or more of the preceding claims, characterized in that the proportion of gas in the adhesive composition is 10-80 per cent by volume, preferably about 30-70%.

**8.** Carrier material having a self-adhesive finish according to one or more of the preceding claims, characterized in that the adhesive composition is composed on the basis of an A-B-A block copolymer, A being polystyrene and B being ethylene, propylene, butylene, butadiene, isoprene or mixtures thereof such as ethylene/ butylene.

**9.** Process for producing carrier material having a self-adhesive finish according to one or more of the preceding claims, characterized in that the hotmelt self- adhesive compositions are reacted under high pressure with inert gases in proportions by volume of 10-80% in a stator/rotor system, the pressure-sensitive adhesive foam produced in this way is passed into the supply nozzle of a melt coating device, and is applied thereby to the carrier material.

**10.** Use of a carrier material having a self-adhesive finish according to one or more of Claims 1 to 7 for medical products.

11. Use according to Claim 10, where the medical products are sticking plasters, fixing plasters, test plasters, rapid dressings, bandages, orthopaedic bandages, wound pads, incision films or colostomy bags.


**Revendications**

1. Matériau de support perméable à l'air avec un apprêt autocollant et une composition autocollante thermofusible appliquée sur toute la surface d'au moins une face, caractérisé en ce que la composition adhésive thermoplastique est expansée et le produit présente une perméabilité à l'air d'au moins 3 $cm^3/cm^2/s$ pour une quantité de composition appliquée d'au moins 20 $g/m^2$.

2. Matériau de support avec un apprêt autocollant selon la revendication 1, caractérisé en ce qu'il présente une perméabilité à l'air de 3 à 150 $cm^3/cm^2/s$, de préférence de 15 à 125 $cm^3/cm^2/s$, en particulier de 25 à 100 $cm^3/cm^2/s$.

3. Matériau de support avec un apprêt autocollant selon la revendication 1 ou 2, caractérisé en ce que la quantité de composition adhésive appliquée est de 30 à 200 $g/m^2$, de préférence de 30 à 160 $g/m^2$.

4. Matériau de support avec un apprêt autocollant selon l'une ou plusieurs des revendications précédentes, caractérisé en ce qu'il présente une perméabilité à la vapeur d'eau d'au moins 100 $g/m^2/24$ h.

5. Matériau de support avec un apprêt autocollant selon la revendication 4, caractérisé en ce qu'il présente une perméabilité à la vapeur d'eau de 100 à 5000 $g/m^2/24$ h, de préférence de 500 à 3000 $g/m^2/24$ h.

6. Matériau de support avec un apprêt autocollant selon l'une ou plusieurs des revendications précédentes, caractérisé en ce que la composition adhésive est expansée avec un gaz inerte tel que l'azote, le dioxyde de carbone, des gaz nobles, des hydrocarbures ou l'air, ou leurs mélanges.

7. Matériau de support avec un apprêt autocollant selon l'une ou plusieurs des revendications précédentes, caractérisé en ce que la proportion de gaz dans la composition adhésive est de 10 à 80 pour cent en volume, de préférence d'environ 30 à 70%.

8. Matériau de support avec un apprêt autocollant selon l'une ou plusieurs des revendications précédentes, caractérisé en ce que la composition adhésive est constituée à base d'un copolymère bloc A-B-A, A représentant le polystyrène et B représentant l'éthylène, le propylène, le butylène, le butadiène, l'isoprène ou leurs mélanges tel que l'éthylène/butylène.

9. Procédé de production d'un matériau de support avec un apprêt autocollant selon l'une ou plusieurs des revendications précédentes, caractérisé en ce que les compositions autocollantes thermofusibles sont mises à réagir sous une pression élevée avec des gaz inertes en proportions volumiques de 10 à 80% dans un système de stator/rotor, la mousse adhésive autocollante ainsi préparée est passée dans l'unité d'alimentation de la buse d'un dispositif de revêtement en fusion et est appliquée sur le matériau de support à partir de celle-ci.

10. Utilisation d'un matériau de support avec un apprêt autocollant selon l'une ou plusieurs des revendications 1 à 7 pour des produits médicaux.

11. Utilisation selon la revendication 10, dans laquelle les produits médicaux sont des sparadraps, des emplâtres de fixation, des emplâtres d'essai, des pansements rapides, des bandes, des bandes et bandages orthopédiques, des tampons vulnéraires, des films d'incision ou des poches pour colostomie.